# EUROPÄISCHE PATENTANMELDUNG

(11) **EP 4 151 110 A1**
(43) Veröffentlichungstag der Anmeldung: **22.03.2023**
(21) Anmeldenummer: 22196142.8
(22) Anmeldetag: 16.09.2022
(51) Int. Cl.: A41C 3/00, A41C 3/04, A41D 13/005

(54) **BÜSTENHALTER MIT TASCHE**

(30) Priorität: 17.09.2021 DE 102021124165; 06.04.2022 DE 102022108260
(71) Anmelder: Radünz, Meike, 13407 Berlin (DE)
(72) Erfinder: Radünz, Sabine, 13407 Berlin (DE)
(74) Vertreter: Dantz, Dirk

(57) **Zusammenfassung**

Die Erfindung betrifft einen Büstenhalter (1) mit einem Körbchen (10, 11) und einem Brustband (20, 21, 22, 23, 24), wobei der Büstenhalter (1) eine Tasche (40, 40') aufweist, die am Körbchen (10, 11) in der Körbchenmitte angeordnet und/oder anordenbar ist, sowie eine Tasche (40, 40'), die an einem Büstenhalter (1) mit einem Körbchen (10, 11) und einem Brustband (20, 21, 22, 23, 24) befestigbar ist.

## Beschreibung

Die Erfindung betrifft einen Büstenhalter mit einem Körbchen und einem Brustband, wobei der Büstenhalter eine Tasche aufweist, die am Körbchen in der Körbchenmitte angeordnet und/oder anordenbar ist, sowie eine Tasche, die an einem Büstenhalter mit einem Körbchen und einem Brustband befestigbar ist.

### Stand der Technik

Still-Büstenhalter (Still-BHs) sind bekannt. Derartige Büstenhalter (BHs) weisen üblicherweise ein aufklappbares Körbchen auf, das es einem Säugling ermöglicht, die Brustwarze der Trägerin des BHs leicht zu erreichen. Gleichzeitig wird die Brust der Trägerin in Position gehalten.

In der Stillzeit werden häufig Kühl- bzw. Wärmeeinlagen verwendet. Wärme entspannt die Brust und bereitet sie auf das Stillen vor. Die Gefäße weiten sich und helfen beim Abfluss von gestauter Milch. Zudem stimuliert Wärme den Milchspendereflex und Milchknötchen lockern sich und können sich auflösen. Eine Kühlung der Brust hilft zum Beispiel bei Brustentzündungen oder beim Milcheinschuss.

Zum Erwärmen der Brust wird ein Waschlappen mit heißem Wasser herangezogen oder es werden bekannte Kälte- bzw. Wärmeeinlage erwärmt und auf die Brust gelegt. Oftmals ist dies den Frauen zu aufwendig, sodass sie es nur kurze Zeit anwenden, was manchmal zur Folge hat, dass die Brust nicht richtig entleert werden kann.

Es ist daher Aufgabe der vorliegenden Erfindung, die genannten Nachteile zu beheben.

Die Aufgabe wird mittels eines Büstenhalters mit Körbchen und einem Brustband nach Anspruch 1 gelöst. Weitere vorteilhafte Ausgestaltungen sind in den Unteransprüchen beschrieben.

Der erfindungsgemäße Büstenhalter weist ein Körbchen und ein Brustband auf. Erfindungsgemäß weist der Büstenhalter eine Tasche auf, die am Körbchen bevorzugt in der Körbchenmitte angeordnet und/oder anordenbar ist. Im Sinne dieser Erfindung ist die Körbchenmitte der Bereich des Körbchens, der sich rund um die Position der Brustwarze anordnet.

In einer optionalen Weiterführung der Erfindung ist die Tasche beabstandet vom unteren Brustband angeordnet. Es gibt in dieser Ausführungsform keinen Punkt, indem sich die Tasche und das Brustband berühren. Die Tasche ist in jedem Punkt beabstandet vom unteren Brustband angeordnet. In einer speziellen Ausführungsform beträgt der Abstand der Tasche zum unteren Brustband mindestens 2 cm, bevorzugt mindestens 3 cm und besonders bevorzugt mindestens 5 cm.

Die Tasche ist dafür vorgesehen, eine Kälte- bzw. Wärmeeinlage aufzunehmen. Die Tasche kann in dem Büstenhalter eingenäht sein, alternativ oder zusätzlich kann die Tasche abnehmbar ausgeführt sein. Eine Kälte- bzw. Wärmeeinlage wird auf maximal 52°C erwärmt und kann daher direkt auf der Haut getragen werden. Die Kälte- bzw. Wärmeeinlage hält die Wärme für ca. 20-30 Minuten

Die Trägerin des erfindungsgemäßen Büstenhalters kann beide Hände zum Halten des zu stillenden Säuglings einsetzen.

In einer weiteren Ausbildung der Erfindung weist die Innenseite des Körbchens an der Körbchenmitte eine Öffnung für die Brustwarze auf. Die Öffnung legt die Brustwarze der weiblichen Brust derart frei, dass ein zu stillender Säugling die Brustwarze erreichen kann.

Der erfindungsgemäße Büstenhalter ist daher auch als herkömmlicher Still-BH wie aus dem Stand der Technik bekannt einsetzbar.

In einer Weiterbildung der Erfindung ist die Öffnung des Körbchens mit einem Stillzugang öffenbar und/oder verschließbar. Der Stillzugang ist als Stoffabdeckung ausgebildet und legt die Öffnung für die Brustwarze der weiblichen Brust für einen zu stillenden Säugling frei bzw. schließt die Öffnung bei Nichtgebrauch. Der erfindungsgemäße Büstenhalter ist daher wie ein herkömmlicher Still-BH einsetzbar.

In einer weiteren Ausgestaltung der Erfindung ist die Tasche abnehmbar ausgestaltet. Bei Nichtgebrauch der Tasche ist der erfindungsgemäße Büstenhalter wie ein herkömmlicher Still-BH einsetzbar.

In einer weiteren Ausbildung der Erfindung weist die Tasche und/oder der Büstenhalter ein Verbindungselement auf, mit dem die Tasche am Körbchen des Büstenhalters lösbar verbindbar ist. Bei Nichtgebrauch ist die Tasche daher von dem Büstenhalter entfernbar, der erfindungsgemäße Büstenhalter kann wie ein herkömmlicher Büstenhalter getragen werden.

In einer Weiterbildung der Erfindung ist das Verbindungselement ein Klettverschluss, ein Clip, ein Knopf, ein Druckknopf, ein Reißverschluss oder ähnliches. Das Verbindungselement ist derart leicht bedienbar, dass die Trägerin des erfindungsgemäßen Büstenhalters die Tasche komfortabel mit einer Hand befestigen bzw. lösen kann. Eine Verbindung mittels Clipverbindung ist leicht manuell herzustellen bzw. wieder zu lösen und gewährleistet gleichzeitig eine sichere Verbindung zwischen Büstenhalter und Tasche. Eine Klammerverbindung mit Haken und Öse funktioniert nach dem Schlüssel-Schloss-Prinzip, wobei jeweils die korrespondierenden Verbindungselemente am Büstenhalter und an der Tasche angeordnet sind.

In einer weiteren Ausführung der Erfindung ist die Tasche in mindestens zwei Punkten mit dem Körbchen verbindbar und/oder verbunden. Die Tasche ist durch mindestens zwei Aufhängepunkte sicher und stabil mit dem Körbchen verbunden. Vor und während des Stillvorgangs wird die Tasche in ihrer korrekten Position gehalten und fixiert.

In einer Weiterbildung der Erfindung weist die Tasche eine Öffnung auf. Mittels der Öffnung kann eine Kühl- bzw. Wärmeeinlage in die Tasche eingeschoben und/oder in der Tasche angeordnet werden.

In einer vorteilhaften Ausgestaltung der Erfindung ist die Öffnung geeignet und dafür vorgesehen, eine Kühl- bzw. Wärmeeinlage in die Tasche einzulegen. Die Tasche ist aus einem dünnen Stoff gefertigt, der die Wärme einerseits gut abgibt, anderseits aber auch die empfindliche Haut an der Brust schützt.

In einer weiteren Ausbildung der Erfindung ist die Tasche auf der Innenseite des Körbchens angeordnet und/oder anordenbar. Die Wirkung einer in der Tasche angeordneten Kühl- bzw. Wärmeeinlage wird daher durch den üblicherweise isolierenden Stoff des Büstenhalters nicht beeinträchtigt.

In einer Weiterbildung der Erfindung überdeckt die dem Körbchen abgewandte Seite der Tasche das Tascheninnere vollständig. Die Haut der Brust kommt daher nicht in Kontakt mit einer in der Tasche angeordneten Kühl- bzw. Wärmeeinlage, die empfindliche Haut der Brust wird vor übermäßiger Kälte und/oder Wärme geschützt.

In einer vorteilhaften Ausgestaltung der Erfindung weist die Tasche einen Gurt auf, an dessen Ende ein Verbindungselement angeordnet ist. Insbesondere ist die Tasche mittels des Verbindungselements lösbar an einem Büstenhalter befestigbar. Bei Nichtgebrauch ist die Tasche daher von dem Büstenhalter entfernbar, der erfindungsgemäße Büstenhalter kann wie ein herkömmlicher Büstenhalter getragen werden.

In einer vorteilhaften Weiterbildung ist der Gurt in der Länge verstellbar. Der Gurt und damit die Tasche ist mittels des verstellbaren Gurtes flexibel an die individuelle Brust der Trägerin der Tasche anpassbar.

Die Aufgabe wird weiterhin mittels der erfindungsgemäßen Tasche zur Befestigung an einem Büstenhalter mit einem Körbchen und einem Brustgurt gelöst. Weitere vorteilhafte Ausführungen der Erfindung sind ebenfalls in den Unteransprüchen dargelegt.

Die erfindungsgemäße Tasche zur Befestigung an einem Büstenhalter mit einem Körbchen und einem Brustgurt weist ein Verbindungselement auf, das dafür vorgesehen und dafür geeignet ist, am Körbchen des Büstenhalters befestigt zu werden. Bei Nichtgebrauch ist die erfindungsgemäße Tasche daher mittels des Befestigungselements von dem Büstenhalter entfernbar, der Büstenhalter kann dann wie ein herkömmlicher Büstenhalter getragen werden.

In einer Weiterbildung der Erfindung ist das Verbindungselement der Tasche dafür vorgesehen und dafür geeignet, die Tasche am Körbchen des Büstenhalters zu befestigen. Das Befestigungselement ist derart am Körbchen des Büstenhalters lösbar befestigbar, dass die am Befestigungselement angeordnete Tasche lösbar mit dem Büstenhalter verbunden ist. Das Befestigungselement ist derart leicht bedienbar, dass die Trägerin des erfindungsgemäßen Büstenhalters die Tasche komfortabel mit einer Hand befestigen bzw. lösen kann.

In einer vorteilhaften Ausgestaltung der Erfindung die Tasche geeignet und dafür vorgesehen, eine Kühl- bzw. Wärmeeinlage aufzunehmen. Die Tasche ist aus einem dünnen Stoff gefertigt, der die Wärme einerseits gut abgibt, anderseits aber auch die empfindliche Haut an der Brust schützt.

In einer vorteilhaften Ausführung der Erfindung weist die Tasche einen Gurt auf. Mittels des Gurtes ist die Tasche an einem Büstenhalter befestigbar.

In einer weiteren Ausbildung der Erfindung ist der Gurt an einem Ende der Tasche befestigt und an seinem anderen Ende ist ein Verbindungselement angeordnet. Insbesondere ist die Tasche mittels des Verbindungselements lösbar an einem Körbchen eines Büstenhalters befestigbar. Bei Nichtgebrauch ist die Tasche daher von dem Büstenhalter entfernbar, der erfindungsgemäße Büstenhalter kann wie ein herkömmlicher Büstenhalter getragen werden.

In einer vorteilhaften Weiterbildung ist der Gurt in der Länge verstellbar. Der Gurt und damit die Tasche ist mittels des verstellbaren Gurtes flexibel an die individuelle Brust der Trägerin der Tasche anpassbar.

Ausführungsbeispiele des erfindungsgemäßen Büstenhalters und der erfindungsgemäßen Tasche sind in den Zeichnungen schematisch vereinfacht dargestellt und werden in der nachfolgenden Beschreibung näher erläutert.

Es zeigen:
- Fig. 1:: Erfindungsgemäßer Büstenhalter
- Fig. 2:: Erfindungsgemäßer Büstenhalter mit nicht lösbar angeordneter Tasche
- Fig. 3 a:: Erfindungsgemäße Tasche zur Aufnahme von Kälte- bzw. Wärmeeinlagen, Rückansicht

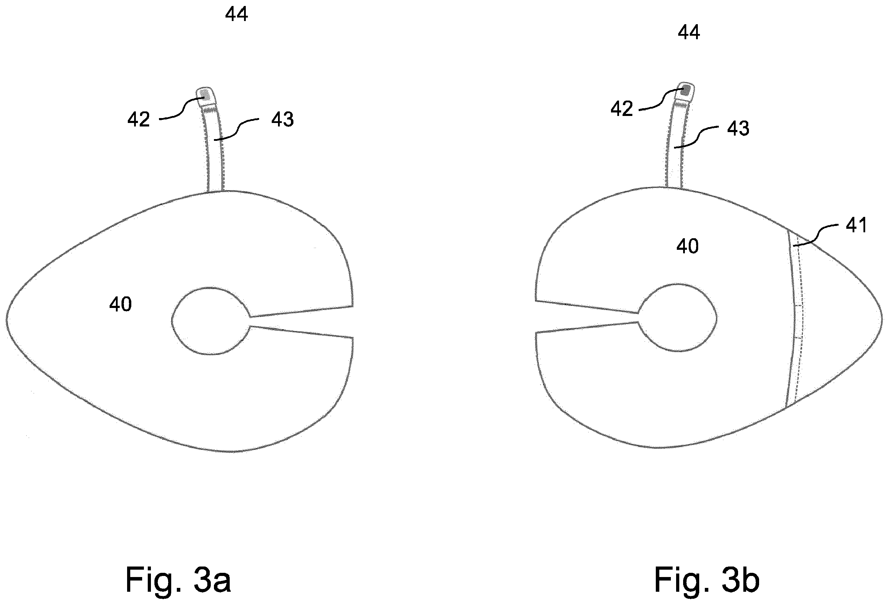

- Fig. 3 b:: Erfindungsgemäße Tasche zur Aufnahme von Kälte- bzw. Wärmeeinlagen, Vorderansicht

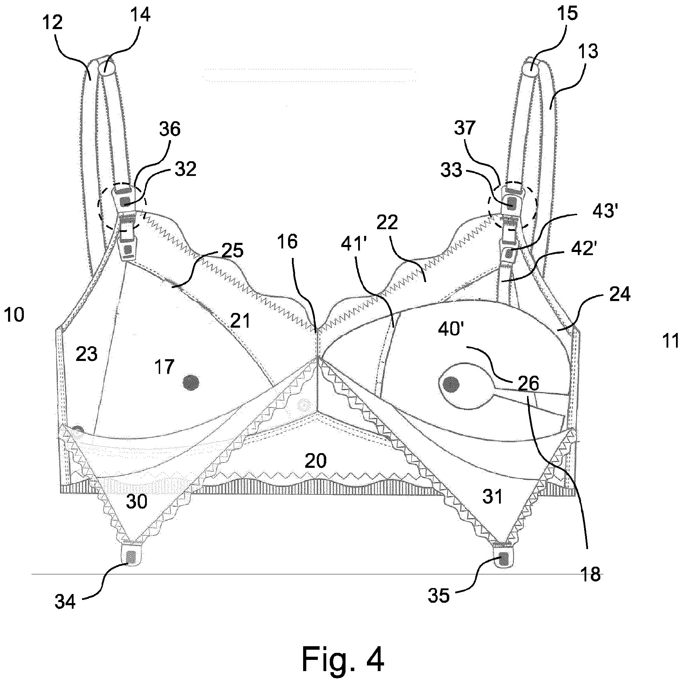

- Fig. 4:: Erfindungsgemäßer Büstenhalter mit lösbar angeordneter Tasche

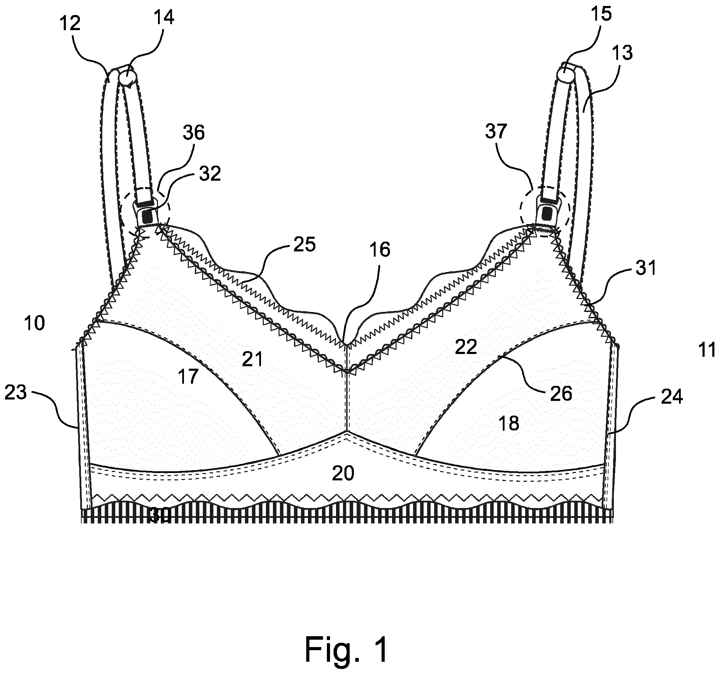

Fig. 1 zeigt eine Vorderansicht eines Ausführungsbeispiels des erfindungsgemäßen Büstenhalters 1. Der Büstenhalter 1 weist ein erstes Körbchen 10 und ein zweites Körbchen 11 sowie das untere Brustband 20, das sich eng unterhalb der Brüste um den Brustkorb anschmiegt, auf. Die Körbchen 10, 11 werden zur Oberseite jeweils von den oberen Brustbändern 21, 22 begrenzt, zu den Außenseiten des Büstenhalters 1 jeweils durch die Seitenteile 23, 24.

Die oberen Brustbänder 21, 22 sowie die Seitenteile 23, 24 weisen jeweils einen vernähten Saum 25 auf. In der Mitte zwischen den Körbchen 10, 11 ist ein Mittelsteg 16 angeordnet. Der Büstenhalter 1 weist ebenfalls einen ersten Träger 12 sowie spiegelsymmetrisch vom ersten Träger 12 angeordnet einen zweiten Träger 13 auf. Die Träger 12, 13 sind mittels der Verstellelemente 14, 15 in der Länge verstellbar.

Die Öffnungen 17, 18 für die Brustwarzen der weiblichen Brust sind im Wesentlichen in der Mitte eines Körbchens 10, 11 angeordnet und werden jeweils von dem unteren Brustband 20, einem oberen Brustband 21, 22 sowie durch jeweils ein Seitenteil 23, 24 begrenzt. Die Brustbänder 20, 21, 22 und die Seitenteile 23, 24 weisen an den Rändern vernähte Säume 25, 27, auf.

Die Öffnungen 17, 18 sind jeweils mittels eines Stillzugangs 30, 31 öffenbar bzw. verschließbar ausgeführt. Ein Stillzugang 30, 31 ist in geschlossenem Zustand des Büstenhalters 1 mittels eines Verschlusselements 36, 37 an jeweils einem Träger 12, 13 befestigt.

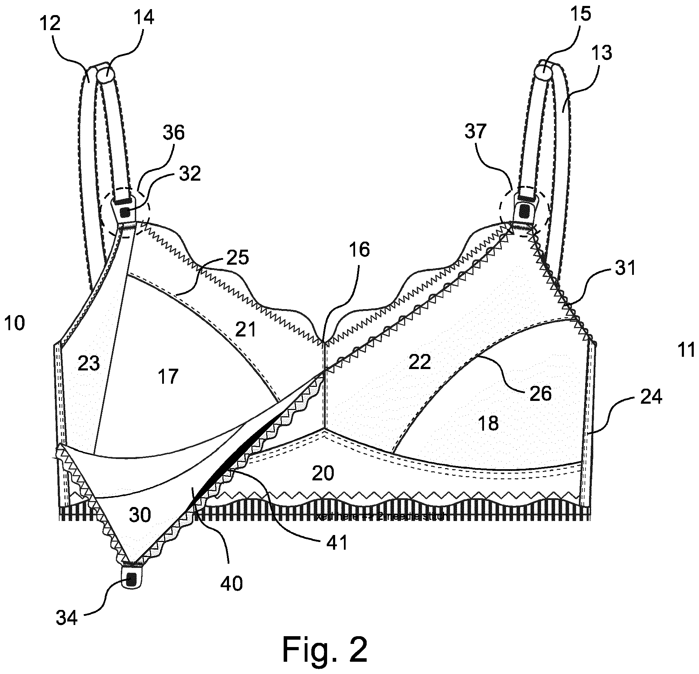

Fig. 2 zeigt ein Ausführungsbeispiel des erfindungsgemäßen Büstenhalters 1, wobei der erste Stillzugang 30 geöffnet der zweite Stillzugang 31 geschlossen ist. Das Verschlusselement 36 des ersten Stillzugangs 30 weist dazu an dem ersten Träger 12 ein erstes Verschließelement 32 auf, der Stillzugang 30 weist das zum ersten Verschließelement 32 korrespondierende zweite Verschließelement 34 auf. In analoger Weise weist das Verschlusselement 37 des zweiten Stillzugangs 31 ein erstes Verschließelement 33 am zweiten Träger 13 auf, der Stillzugang 31 weist das zum ersten Verschließelement 33 korrespondierende zweite Verschließelement 35 auf. Die Verschlusselemente 36, 37 sind in diesem Ausführungsbeispiel Clipverbindungen, möglich ist auch z.B. eine Klemmverbindung, einer Klammerverbindung mit Haken und Ösen, oder auch eine Klettverbindung.

In dem ersten Stillzugang 30 ist die erfindungsgemäße Tasche 40 nicht abnehmbar angeordnet. Die Tasche 40 selbst weist die Öffnung 41 auf, mittels der eine Kälte- bzw. Wärmeeinlage derart in die Tasche 40 eingeführt werden kann, dass die Kälte- bzw. Wärmeeinlage in der Tasche 40 angeordnet ist. Die Tasche 40 ist in geschlossenem Zustand des ersten Stillzugangs 30 auf der Innenseite des ersten Körbchens 10 angeordnet. Die Tasche 40 ist aus einem dünnen Stoff gefertigt, der die Wärme einerseits gut abgibt, anderseits aber auch bei einer in der Tasche 40 angeordneten Kälte- bzw. Wärmeeinlage die empfindliche Haut der Brust schützt.

Fig. 3 zeigt eine abnehmbare Tasche 44, die an einem Büstenhalter 1 angeordnet werden kann. Die abnehmbare Tasche 44 weist neben der Tasche 40 den in der Länge verstellbaren Gurt 43 auf, der an einem Ende ein Verbindungselement 42 aufweist, am dem Verbindungselement 42 gegenüberliegenden Ende ist die Tasche 40 angeordnet (Fig. 3 a). Mittels des Verbindungselements 42 ist die Tasche lösbar an einem Büstenhalter 1 befestigbar.

Das Verbindungselement 42 ist ebenfalls eine Clipverbindung, möglich ist auch z.B. eine Klemmverbindung, einer Klammerverbindung mit Haken und Ösen, oder auch eine Klettverbindung sowie ein Reißverschluss. Die Tasche 40 weist die Öffnung 41 auf (Fig. 3 b), die dafür vorgesehen ist, eine Kälte- bzw. Wärmeeinlage in der Tasche 40 anzuordnen.

Eine abnehmbar ausgeführte Tasche 44' (s. Fig. 3) zur Aufnahme einer Kälte- bzw. Wärmeeinlage, angeordnet in einem Büstenhalter 1, zeigt Fig. 4. Die Tasche 40' ist auf der Innenseite der Körbchenmitte des zweiten Körbchens 11 angeordnet und liegt direkt auf der Haut der Trägerin des Büstenhalters 1 auf. Die Tasche 40' ist mittels des Verbindungselements 42' des Gurtes 43' am ersten Verschließelement 33 des Büstenhalters 1 befestigt. Die Tasche 40' ist durch das Verbindungselement 42' und den an dem unteren Brustband 20 befestigten Stillzugang 31 mit dem zweiten Körbchen 11 verbunden.

### BEZUGSZEICHENLISTE

- 1: Büstenhalter
- 10: Erstes Körbchen
- 11: Zweites Körbchen
- 12, 13: Träger
- 14, 15: Verstellelement des Trägers
- 16: Mittelsteg
- 17, 18: Öffnung für Brustwarze
- 20: Unteres Brustband
- 21, 22: Oberes Brustband
- 23, 24: Seitenteil
- 25, 26, 28: Naht/Saum
- 30, 31: Stillzugang
- 32, 33: Erstes Verschließelement des Stillzugangs
- 34, 35: Zweites Verschließelement des Stillzugangs
- 36, 37: Verschlusselement des Stillzugangs
- 40, 40': Tasche
- 41, 41': Öffnung der Tasche
- 42 ,42': Verbindungselement / Befestigungselement der Tasche
- 43, 43': Gurt
- 44: Abnehmbare Tasche
- BW: Brustwarze

## Patentansprüche

1. Büstenhalter (1) mit einem Körbchen (10, 11) und einem Brustband (20, 21, 22, 23, 24)
**dadurch gekennzeichnet, dass**
der Büstenhalter (1) eine Tasche (40, 40') aufweist, die am Körbchen (10, 11) in der Körbchenmitte angeordnet und/oder anordenbar ist.

2. Büstenhalter (1) mit einem Körbchen (10, 11) und einem Brustband (20, 21, 22, 23, 24) nach Anspruch 1
**dadurch gekennzeichnet, dass**
die Innenseite des Körbchens (10, 11) an der Körbchenmitte eine Öffnung (17, 18) für die Brustwarze (BW) aufweist,
wobei die Öffnung (17, 18) des Körbchens (10, 11) mit einem Stillzugang (30, 31) verschließbar ist.

3. Büstenhalter (1) mit einem Körbchen (10, 11) und einem Brustband (20, 21, 22, 23, 24) nach Anspruch 1 oder 2
**dadurch gekennzeichnet, dass**
die Tasche (40, 40') abnehmbar ausgestaltet ist.

4. Büstenhalter (1) mit einem Körbchen (10, 11) und einem Brustband (20, 21, 22, 23, 24) nach einem oder mehreren der vorhergehenden Ansprüche
**dadurch gekennzeichnet, dass**
die Tasche (40, 40') und/oder der Büstenhalter (1) ein Verbindungselement (42, 42') aufweist, mit dessen Hilfe die Tasche (40, 40') am Körbchen (10, 11) des Büstenhalters (1) lösbar verbindbar ist,
wobei das Verbindungselement (42, 42') ein Klettverschluss, ein Clip, ein Knopf, ein Druckknopf, ein Reißverschluss oder ähnliches ist.

5. Büstenhalter (1) mit einem Körbchen (10, 11) und einem Brustband (20, 21, 22, 23, 24) nach einem oder mehreren der vorhergehenden Ansprüche
**dadurch gekennzeichnet, dass**
die Tasche (40, 40') in mindestens zwei Punkten mit dem Körbchen (10, 11) verbindbar und/oder verbunden ist.

6. Büstenhalter (1) mit einem Körbchen (10, 11) und einem Brustband (20, 21, 22, 23, 24) nach einem oder mehreren der vorhergehenden Ansprüche
**dadurch gekennzeichnet, dass**
die Tasche (40,40') eine Öffnung (41, 41') aufweist, die dafür geeignet und vorgesehen ist, eine Kälte- bzw. Wärmeeinlage in diese einzulegen.

7. Büstenhalter (1) mit einem Körbchen (10, 11) und einem Brustband (20, 21, 22, 23, 24) nach einem oder mehreren der vorhergehenden Ansprüche
**dadurch gekennzeichnet, dass**
die Tasche (40, 40') auf der Innenseite des Körbchens (10, 11) angeordnet und/oder anordenbar ist.

8. Büstenhalter (1) mit einem Körbchen (10, 11) und einem Brustband (20, 21, 22, 23, 24) nach einem oder mehreren der vorhergehenden Ansprüche,
**dadurch gekennzeichnet, dass**
die dem Körbchen (10, 11) abgewandte Seite der Tasche (40, 40') das Tascheninnere vollständig überdeckt.

9. Büstenhalter (1) mit einem Körbchen (10, 11) und einem Brustband (20, 21, 22, 23, 24) nach einem oder mehreren der vorhergehenden Ansprüche,
**dadurch gekennzeichnet, dass**
die Tasche (40, 40') einen Gurt (43, 43') aufweist, an dessen Ende ein Verbindungselement (42, 42') angeordnet ist,
wobei der Gurt (43, 43') in der Länge verstellbar ist.

10. Tasche (40, 40') zur Befestigung an einem Büstenhalter (1) mit einem Körbchen (10, 11) und einem Brustband (20, 21, 22, 23, 24) nach einem oder mehreren der vorhergehenden Ansprüche
**dadurch gekennzeichnet, dass**
die Tasche (40, 40') ein Verbindungselement (42, 42') aufweist, das dafür vorgesehen und dafür geeignet ist, am Körbchen (10, 11) des Büstenhalters (1) befestigt zu werden,
wobei das Verbindungselement (42, 42') der Tasche (40, 40') dafür vorgesehen und dafür geeignet ist, die Tasche am Körbchen (10, 11) des Büstenhalters (1) zu befestigen, und
wobei die Tasche (40, 40') der Aufnahme eines Wärme- und/oder Kältepads dient.

11. Tasche (40, 40') zur Befestigung an einem Büstenhalter (1) mit einem Körbchen (10, 11) und einem Brustband (20, 21, 22, 23, 24) nach Anspruch 10,
**dadurch gekennzeichnet, dass**
die Tasche (40, 40') einen Gurt (43, 43') aufweist,
wobei der Gurt (43, 43') an einem Ende an der Tasche (40, 40') befestigt ist und an seinem anderen Ende ein Verbindungselement (42, 42') aufweist, und
wobei der Gurt (43, 43') in seiner Länge verstellbar ist.
